# EUROPEAN PATENT APPLICATION

(11) **EP 2 545 891 A1**
(43) Date of publication of application: **16.01.2013**
(21) Application number: 11174041.1
(22) Date of filing: 14.07.2011
(51) Int. Cl.: A61F 15/00, A61F 13/00, A61K 9/70

(54) **Patch package**

(71) Applicant: Nitto Denko Corporation, Osaka 567-8680 (JP)
(72) Inventor: Suzuki, Minoru, Osaka 567-8680 (JP); Yamamoto, Keiji, Osaka 567-8680 (JP); Konno, Masakatsu, Osaka 567-8680 (JP); Yaegashi, Hiroyuki, Osaka 567-8680 (JP); Izaki, Toshiharu, Osaka 567-8680 (JP); Sakuraba, Ryohei, Osaka 567-8680 (JP)
(74) Representative: Hopkin, Tobias J.B.

(57) **Abstract**

The present invention relates to a patch package having a patch and a packaging film sandwiching the patch, wherein the packaging film is tightly sealed in two or more flat heat-sealed parts, and respective adjacent two or more flat heat-sealed parts are separated across a non-sealed part.

## Description

### TECHNICAL FIELD OF THE INVENTION

The present invention relates to a patch package wherein a patch is packed with a packaging film. More particularly, the present invention relates to a patch package wherein packaging films facing across a patch are hermetically-sealed in a flat heat-sealed part.

### BACKGROUND OF THE INVENTION

In recent years, various patchs have been developed to protect and heal a wound, a pressure ulcer, a thermal burn ulcer, a peel off-wound and the like on the human skin. In addition, as a means of administering a drug to a body, a method comprising adhering a patch containing a drug to a body to allow transdermal absorption of the drug from the skin has been developed. Such a drug-containing patch (hereinafter sometimes to be referred to as a patch preparation) comprises a support made of a plastic film such as polyester, polyethylene and the like or a non-woven fabric, an adhesive layer containing a transdermally absorbable drug, which is laminated on one surface of the support, and a coating material covering an exposed surface of the adhesive layer. Generally, a patch and a patch preparation are indivisually packed (tightly sealed) in a package made of a packaging film, for the purpose of protecting the patch, preventing volatilization of the contained drug and/or avoiding influences of humidity, oxygen etc. on the drug (decomposition, oxidation etc.) and the like.

Patches are generally packed (tightly sealed) individually by a heat sealing treatment of a packaging film surronding the patch by a packaging machine. However, depending on the kind of a packaging film and a packaging method, the heat sealing treatment may not be sufficient, which causes a problem of pinholes sometimes developed in the part subjected to the heat sealing treatment (hereinafter sometimes to be referred to as a heat-sealed part).

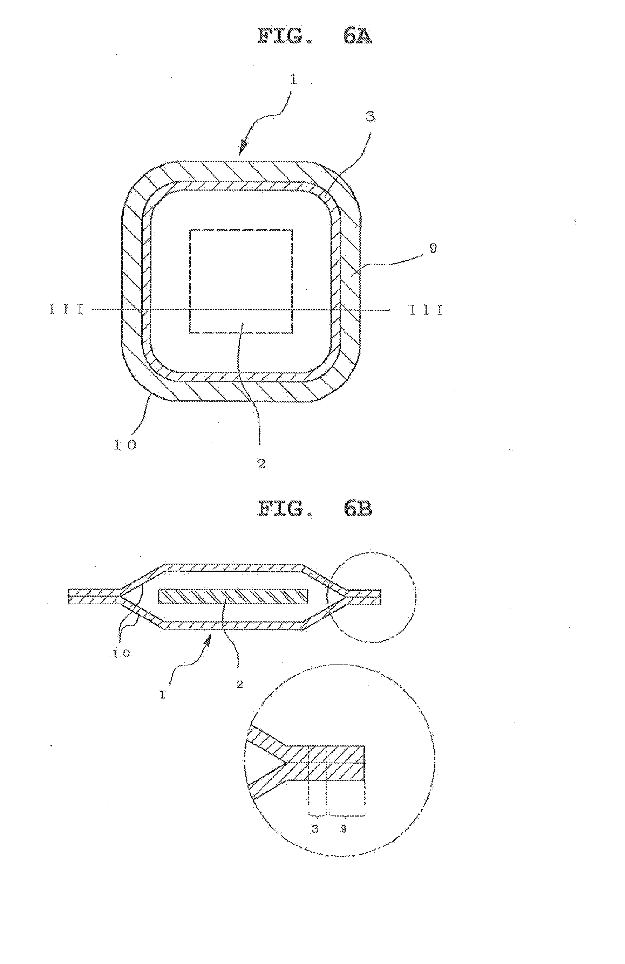

To solve the above-mentioned problem, for example, JP-A-2006-44793 proposes a patch package wherein an embossed heat-sealed part and a flat heat-sealed part in a packaging film surround the periphery of the patch, as shown in Fig. 6A and Fig. 6B. However, when the seal width of the flat heat-sealed part is increased in an attempt to decrease development of pinholes and improve air-tightness, the possibility of developing air bubbles in the flat heat-sealed part becomes high to possibly degrade the appearance thereof.

### SUMMARY OF THE INVENTION

The present invention aims to solve the above-mentioned problems, and provides a patch package which is free of pinholes, superior in air-tightness, and has a good appearance of a flat heat-sealed part.

The present inventors have conducted intensive studies of the aforementioned problems and found a particular effect of improved air-tightness by providing, in a packaging film sandwiching a patch, at least two flat heat-sealed parts in a pattern surrouding the patch, and providing a non-sealed part between the adjacent flat heat-sealed parts, as compared to when a seal width of the flat heat-sealed part is simply increased. In addition, they have found that the non-sealed part acts as an escape of bubbles, that may be generated during formation of the flat heat-sealed part, to decrease the sealing defect in the flat heat-sealed part, whereby a superior flat heat-sealed part free of bubbles can be obtained. The present invention has been completed based on these findings.
Accordingly, the present invention provides the following.

[1] A patch package comprising a patch and a packaging film sandwiching the patch, wherein the packaging film is tightly sealed in two or more flat heat-sealed parts, and respective adjacent flat heat-sealed parts of the two or more flat heat-sealed parts are separated across a non-sealed part.
[2] The patch package of the above-mentioned [1], further comprising an embossed heat-sealed part outside the outermost flat heat-sealed part of the two or more flat heat-sealed parts.
[3] The patch package of the above-mentioned [2], wherein the outermost flat heat-sealed part and the embossed heat-sealed part are separated across a non-sealed part.
[4] The patch package of any one of the above-mentioned [1] - [3], wherein the two or more flat heat-sealed parts, or the two or more flat heat-sealed parts and the embossed heat-sealed part form a pattern surrounding the patch.
[5] The patch package of any one of the above-mentioned [1] - [4], wherein the patch is sandwiched between two packaging films, and the two or more flat heat-sealed parts, or the two or more flat heat-sealed parts and the embossed heat-sealed part form a pattern surrounding the entire periphery of the patch.
[6] The patch package of any one of the above-mentioned [1] - [4], wherein the patch is sandwiched by a double-folded packaging film, and the two or more flat heat-sealed parts, or the two or more flat heat-sealed parts and the embossed heat-sealed part are not formed in a bent part of the packaging film.
[7] The patch package of any one of the above-mentioned [1] - [6], wherein each of the two or more flat heat-sealed parts has a band-like shape.
[8] The patch package of the above-mentioned [7], wherein the band-like flat heat-sealed parts have a band width of 0.4 mm - 1.0 mm.
[9] The patch package of any one of the above-mentioned [1] - [8], wherein the non-sealed part has a band-like shape and has a band width of 1.5 mm - 10 mm.
[10] The patch package of any one of the above-mentioned [1] - [9], having three flat heat-sealed parts, or three flat heat-sealed parts and an embossed heat-sealed part.
[11] The patch package of any one of the above-mentioned [1] - [10], wherein the patch is a patch preparation containing a drug.

The patch package of the present invention is free of pinholes, superior in air-tightness, and has a good appearance of the flat heat-sealed part. In addition, the patch package provides an effect of high suppression of oxidation and decomposition for a drug easily decomposed by oxygen or moisture.
Furthermore, in the patch package of the present invention, each flat heat-sealed part can be certainly formed at a uniform seal width. Therefore, it affords an effect of remarkable reduction of percent defective of flat heat sealing in industrial production of the patch package.
In the patch package of the present invention, moreover, even when one of the two or more flat heat-sealed parts develops pinholes and the like, the remaining flat heat-sealed part ensures sufficient air-tightness. Thus, it affords an effect of remarkable reduction of percent defective of flat heat sealing in industrial production of the patch package.

### BRIEF DESCRIPTION OF THE DRAWINGS

Fig. 1A is a plane view showing embodiment 1 of the present invention.
Fig. 1B is a sectional view along I-I line in Fig. 1A.
Fig. 2A is a plane view showing embodiment 2 of the present invention.
Fig. 2B is a sectional view along II-II line in Fig. 2A.
Fig. 3 is a plane view showing embodiment of the present invention.
Fig. 4 is a plane view showing embodiment 4 of the present invention.
Fig. 5A is a plane view showing embodiment 5 of the present invention.
Fig. 5B is a sectional view along IV-IV line in Fig. 5A.
Fig. 6A is a plane view showing a packaging structure of a conventional patch package.
Fig. 6B is a sectional view along III-III line in Fig. 6A.
Fig. 7 shows the relationship between the preservation period and water content of patches tightly sealed in the patch packages of Examples and Comparative Examples.
In the Figures, 1 is a package, 2 is a patch, 3 is a first flat heat-sealed part, 4 is a second flat heat-sealed part, 5 is a third flat heat-sealed part, 6 is a first non-sealed part, 7 is a second non-sealed part, 8 is a third non-sealed part, 9 is an embossed heat-sealed part, and 10 is a packaging film.

### DETAILED DESCRIPTION OF THE INVENTION

The present invention is explained in detail in the following.
The patch package of the present invention comprises a patch and a packing film sandwiching the patch, and has a structure wherein the packaging film is tightly sealed in two or more flat heat-sealed parts. Two packaging films having approximately the same shape may be placed on top of each other and a patch may be insetted between the two packaging films. Alternatively, one packaging film may be folded in two and a patch may be insetted therebetween. In the present invention, the flat heat-sealed part is where a packaging film is flat heat sealed around a patch, and is generally formed in a pattern surrounding the patch. For example, when a patch is packed by being sandwiched between two packaging films, the flat heat-sealed part is formed in a pattern surrounding the entire periphery of the patch. Particularly, when a package having a quadrate plane shape is to be obtained, a flat heat-sealed part is formed in two packaging films on four sides of the periphery of the patch (four-sided seal). In addition, when a patch is sandwiched by a double-folded packaging film, and the package has a quadrate plane shape, a flat heat-sealed part is formed on three sides of the packaging film except the bent part of the film (three-sided seal). The four-sided seal is advantageous in that it can control the insersion position of a patch when it is sandwiched between the packaging films.

The patch package of the present invention generally has at least two flat heat-sealed parts, preferably 2 - 5 parts and particularly preferably 2 or 3 parts, from the aspect of easy production. While the shape of the flat heat-sealed part (the shape of flat heat-sealed part on the flat plane when the package is seen in a planar view) is not particularly limited, it is preferably a band-like shape. In the case, the width of the flat heat-sealed part (band width) is generally 0.4 mm - 1.0 mm, preferably 0.4 mm - 0.6 mm. When the width (band width) is less than 0.4 mm, the effect of preventing development of pinholes becomes low. When the width exceeds 1.0 mm, the pressure in the heat sealing treatment is dispersed during formation of the flat heat-sealed part and the heat sealing treatment becomes incomplete. As a result, pinholes may be developed, and air bubbles easily enter into the flat heat-sealed part, which is not preferable for the appearance thereof. While the width of the band-like flat heat-sealed part may be uniform or vary, it is preferably uniform. In addition, in two or more band-like flat heat-sealed parts, the width (band width) and pattern of the flat heat-sealed parts may be same or different, and they are preferably the same.

The patch package of the present invention has two or more flat heat-sealed parts, and respective adjacent flat heat-sealed parts are separated across a non-sealed part. With a non-sealed part between the adjacent flat heat-sealed parts, air bubbles developed when forming the flat heat-sealed parts can be released into the non-sealed part, and the flat heat-sealed part can be formed beautifully. While the shape of the non-sealed part (the shape of non-sealed part on the flat plane when the package is seen in a planar view) is not particularly limited, it is preferably a band-like shape. In this case, the width (band width) of the non-sealed part is generally 1.5 mm - 10 mm, preferably 2.0 mm - 10 mm. When the width (band width) of the non-sealed part is not less than 1.5 mm, a patch contained in the package has enhanced preservability. In addition, when the width is not more than 10 mm, a package having good handling property which uses a saved amount of the packaging film can be obtained. When two or more non-sealed parts are present, the width (band width) and pattern of respective non-sealed parts may be the same or different, and preferably the same.

The packaging structure of the present invention may have an embossed heat-sealed part on the circumference of the package. That is, it may have a structure wherein an embossed heat-sealed part is present at the outside of the outermost flat heat-sealed part (that is, the flat heat-sealed part at the farthest position from the patch) in the packaging films sandwiching the patch. The embossed heat-sealed part is a heat-sealed part having concaves and convexes. Examples of the concaves and convexes include patterns such as general straw mat pattern, lattice pattern, straight line pattern, wave shape pattern, point-like pattern and the like. To obtain an appropriate seal strength, a combination of a straw mat pattern and a straight line pattern is preferable. The pitch of the above-mentioned pattern can be set within the range of 0.2 mm - 2 mm, preferably 0.4 mm - 0.8 mm, in the case of, for example, a straw mat pattern. When the pitch of the straw mat pattern is narrower than 0.2 mm, molding using a heat block metal mold for forming an embossed heat-sealed part becomes difficult. When the pitch is wider than 2 mm, pinholes are easily developed and the appearance of the resulting packaging structure is not beautiful. The embossed heat-sealed part is also generally formed in a pattern surrounding the patch. When, for example, a patch is packed by being sandwiched between two packaging films, the embossed heat-sealed part is also formed at the outside of the outermost flat heat-sealed part in a pattern surrounding the entire periphery of the patch.

When the above-mentioned embossed heat-sealed part is present, a non-sealed part may be present between the outermost flat heat-sealed part and the embossed heat-sealed part to separate them. While the shape of the non-sealed part is not particularly limited, a band-like shape is preferable. In this case, the width (band width) of the non-sealed part is generally 1.5 mm - 10 mm, preferably 2.0 mm - 10 mm. When the width of the non-sealed part is not less than 1.5 mm, a package has enhanced preservability for a patch contained in the package. In addition, when the width is not more than 10 mm, a package having good handling property which uses a saved amount of the packaging film can be obtained.
The embossed heat-sealed part is preferably provided at the outer periphery of the package.

The shape of the patch package (planar shape) of the present invention is not particularly limited and, for example, a quadrate (square, rectangle etc.), a polygon (triangle, hexagon etc.), a circular shape, an ellipse, other figures and the like can be mentioned. The shape of the patch package (planar shape) and the shape of the patch to be packed (planar shape) may be the same or different, and can be appropriately selected as long as the tight-sealing property of the package can be maintained.

A preferable embodiment of the patch package of the present invention is explained in detail in the following by referring to the drawings. However, the following explanation is merely illustrative, and the present invention, application thereof and use thereof are not limited thereby. In the drawings, the size of each part in the drawings, the size ratio between respective parts and the like do not necessarily match with those of the actual parts.

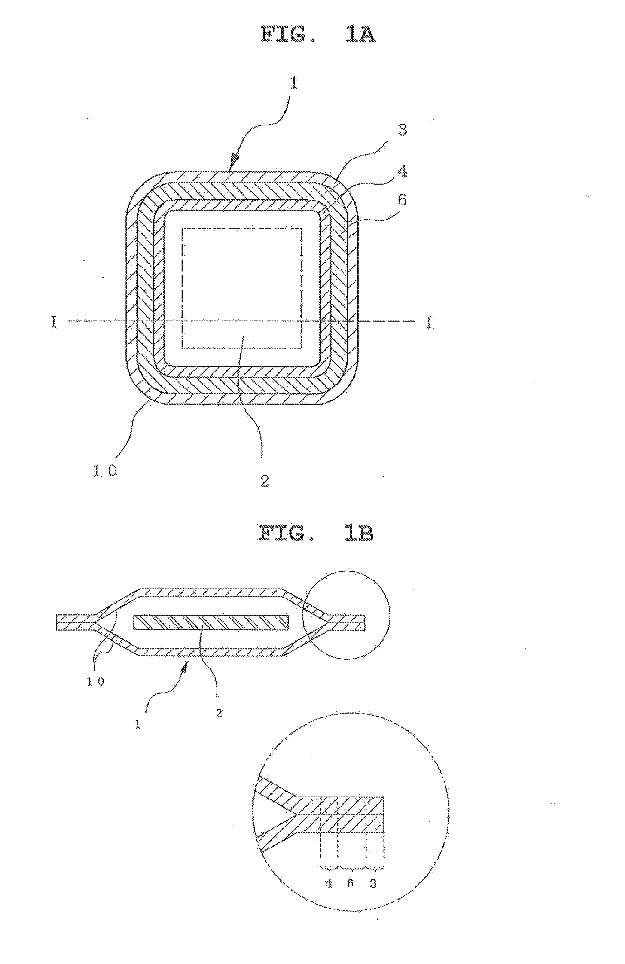

One embodiment of the patch package of the present invention is shown in Fig. 1A and Fig. 1B. A patch 2 is sandwiched between two packaging films 10, and the two packaging films 10 are flat heat sealed around the patch 2 to surround the patch 2, whereby the first flat heat-sealed part 3 and the second flat heat-sealed part 4 are formed. In other words, the patch 2 is packed in package 1 tightly sealed with double flat heat-sealed parts. Here, the first flat heat-sealed part 3 and second flat heat-sealed part 4 are formed at given intervals, and a first non-sealed part 6 is provided between the first flat heat-sealed part 3 and the second flat heat-sealed part 4. That is, the first flat heat-sealed part 3 and the second flat heat-sealed part 4 are separated across the first non-sealed part 6.

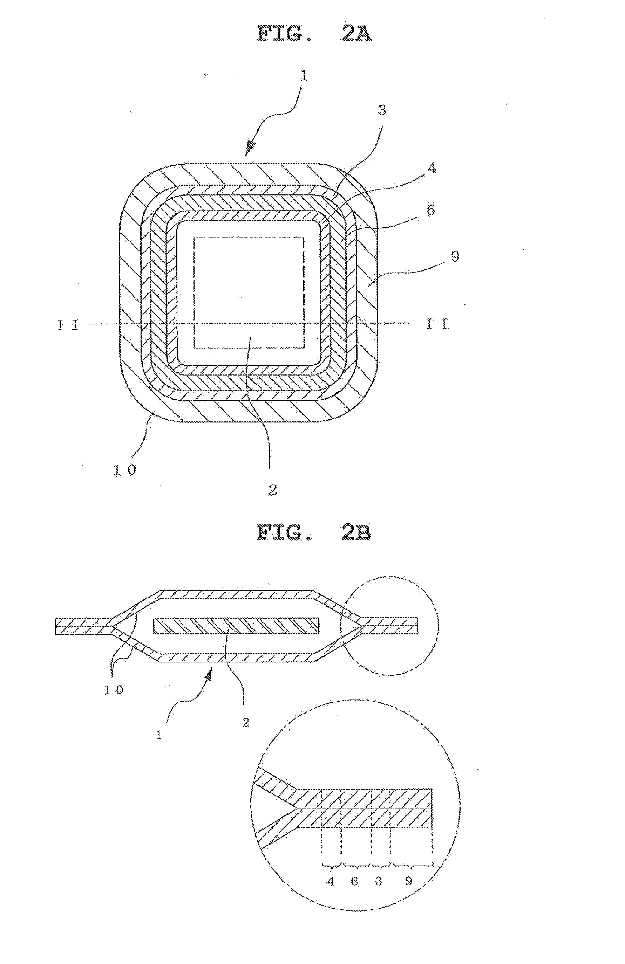

Another embodiment of the patch package of the present invention is shown in Fig. 2A and Fig.2B. The package 1 of this embodiment further has an embossed heat-sealed part 9 in the outer periphery of the package. That is, the package is different from the patch package shown in Fig. 1A and Fig. 1B in that it has an embossed heat-sealed part 9 outside a flat heat-sealed part at the most distant position from patch 2 (first flat heat-sealed part 3). A non-sealed part does not exist between the first flat heat-sealed part 3 and the embossed heat-sealed part 9, and the first flat heat-sealed part 3 and the embossed heat-sealed part 9 are continuously formed. In the patch package of this embodiment, since the outer periphery of the package is the embossed heat-sealed part 9, the package can be opened easily with hand by tearing the outer periphery of the package. In addition, the embossed heat-sealed part 9 may have a pattern (straw mat pattern, lattice pattern, straight line pattern, wave shape pattern, point-like pattern etc.), and the pattern makes a more beautiful appearance of the package.

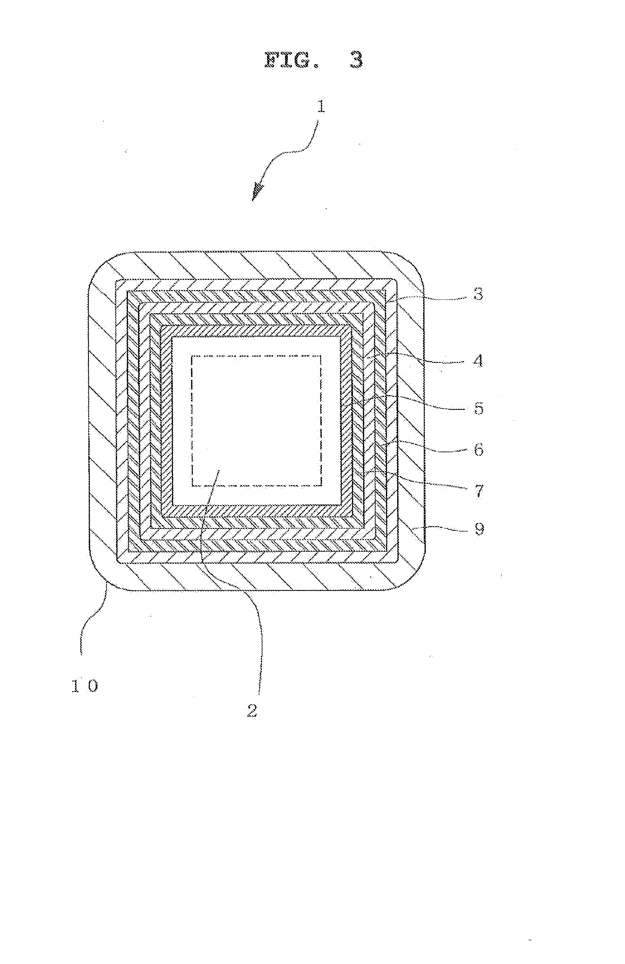

Another embodiment of the patch package of the present invention is shown in Fig. 3. The package 1 of this embodiment is different from the patch package shown in Fig. 2A and Fig. 2B in that it further has a third flat heat-sealed part 5 on the side of the package central part of the second flat heat-sealed part 4 and a second non-sealed part 7 between the second flat heat-sealed part 4 and the third flat heat-sealed part 5. That is, an embossed heat-sealed part 9, a first flat heat-sealed part 3, a first non-sealed part 6, a second flat heat-sealed part 4, a second non-sealed part 7 and a third flat heat-sealed part 5 are sequentially formed from the outer periphery to the central part of the package. In the patch package of this embodiment, the preserving property of a patch 2, which is the content, can be enhanced, since the package is tightly sealed by triple flat heat-sealed parts (first flat heat-sealed part 3, second flat heat-sealed part 4 and third flat heat-sealed part 5) and an embossed heat-sealed part 9.

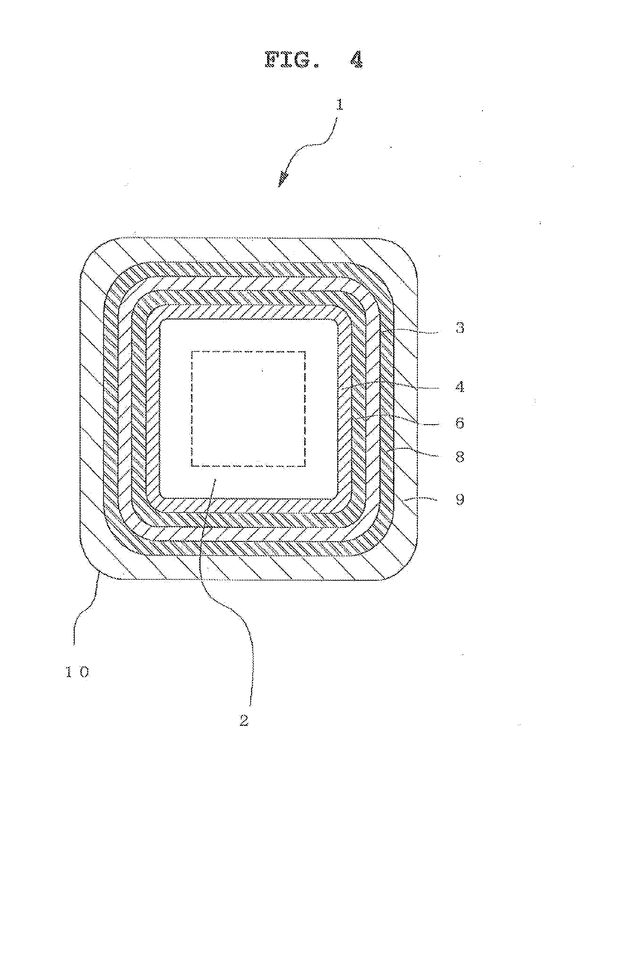

Another embodiment of the patch package of the present invention is shown in Fig. 4. The package 1 of this embodiment is different from the patch package shown in Fig. 2A and Fig. 2B in that it has a non-sealed part (third non-sealed part 8) between the first flat heat-sealed part 3 and the embossed heat-sealed part 9. That is, an embossed heat-sealed part 9, a third non-sealed part 8, a first flat heat-sealed part 3, a first non-sealed part 6, and a second flat heat-sealed part 4 are sequentially formed from the outer periphery to the central part of the package. In the patch package of this embodiment, the package is tightly sealed by the double flat heat-sealed parts, and the embossed heat-sealed part 9 in the outer periphery of the package is separated from the first flat heat-sealed part 3 by the presence of the third non-sealed part 8. With the third non-sealed part 8, the first flat heat-sealed part 3 can be formed without an influence of the embossed heat sealing 9. As a result, the first flat heat-sealed part 3 can secure stronger sealing. In addition, since air bubbles can be easily released from the first flat heat-sealed part 3, the package can be finished more beautifully.

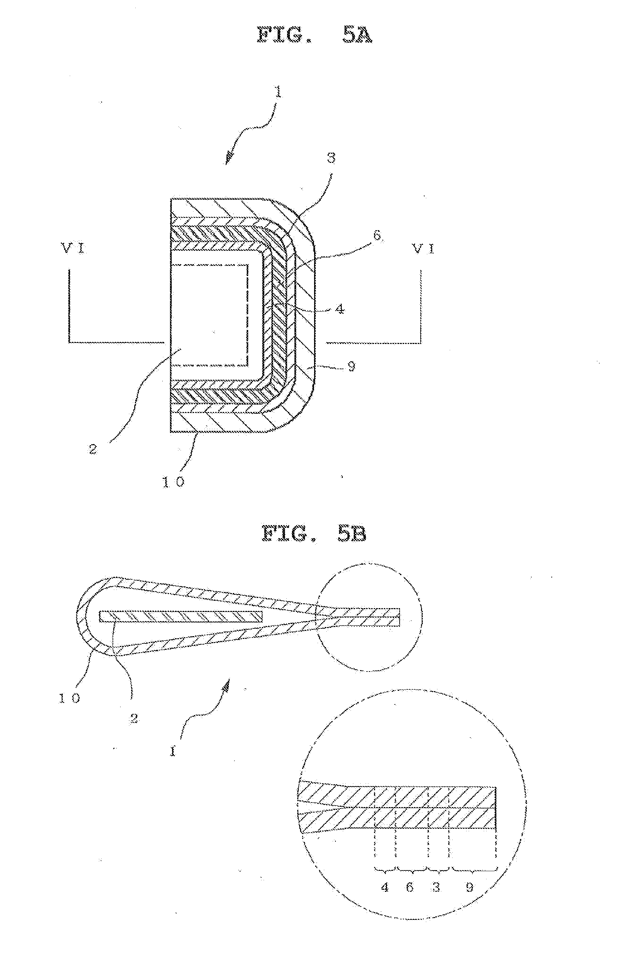

Another embodiment of the patch package of the present invention is shown in Fig. 5A and Fig. 5B. The package 1 of this embodiment is comprised of a double-folded packaging film 10, and an embossed heat-sealed part 9, a first flat heat-sealed part 3, a first non-sealed part 6 and a second flat heat-sealed part 4 are sequentially formed from the outer periphery to the central part of the package 1, on the three sides except the side where the bent part of the packaging film 10 is formed. The bent part of the double-folded packaging film 10 may have roundness except the end to be heat sealed, as shown in Fig. 5B. In this embodiment, the package 1 packing the patch 2 is tightly sealed by the bent part, and the double flat heat-sealed parts and the embossed heat-sealed part, which are formed to surround the patch 2 except the bent part, in the packaging film 10.

In the present invention, the method of a heat sealing treatment is not particularly limited, and a known method can be used. The method of the heat sealing treatment is generally divided into a rotation method, a box motion method and a butt-sealing method, according to the movement of the main spindle.

A patch package of the present invention having a quadrate planar shape can be produced by using a general four-sided seal packaging machine.

One example of the method of producing a patch package by performing a heat sealing treatment according to the rotation method by using a general four-sided seal packaging machine is as follows. First, two long packaging films are placed to face each other, and a patch is inserted between these packaging films to be sandwiched in the vertical direction. Then, the both ends in the longitudinal direction of the two layered packaging films are heated by a heat block. The heated both ends are heat sealed by pressure-bonding with a flat roll having a given width and free of concaves and convexes to form a flat heat-sealed part. By a heat sealing treatment of a little outside of the flat heat-sealed part formed by pressure-bonding with the same flat roll, a second flat heat-sealed part can be formed. By repeating a similar operation, flat heat-sealed parts can be formed in triplicate, quadruplicate or more. By changing the width of the roll to be used, moreover, the width of the flat heat-sealed part can be changed. Furthermore, a little outside of the outermost heat-sealed part formed is heat sealed by pressure-bonding with a roll having a pattern such as a straw mat pattern and the like to form an embossed heat-sealed part (whereby a heat-sealed part is completed on the both ends). Thereafter, a heat sealing treatment is performed by pressure-bonding a metal mold bar integrally having a flat heat sealing treatment surface free of concaves and convexes and a flat heat sealing treatment surface having a pattern such as a straw mat pattern and the like in a direction orthogonal to the longitudinal direction of the packaging film, so that the flat heat-sealed part will be formed inside and the embossed heat-sealed part will be formed outside, whereby a flat heat-sealed part and an embossed heat-sealed part are simultaneously formed (that is, heat-sealed parts orthogonal to the above-mentioned both ends are formed). Thereafter, the packaging film is cut such that each package contains one patch to complete four-sided seal packages.

One example of the method of producing a patch package by performing a heat sealing treatment according to the butt-sealing method by using a general four-sided seal packaging machine is as follows. First, two packaging films are placed to face each other, and a patch is inserted between these packaging films to be sandwiched in the vertical direction. Then, a heat sealing treatment is performed by heating and pressure-bonding a heat block metal mold capable of simultaneously forming heat-sealed parts on 4 sides (having a flat heat sealing treatment surface free of concaves and convexes, and a heat sealing treatment surface having a pattern such as a straw mat pattern etc.) against a packaging film, whereby an embossed heat-sealed part and a flat heat-sealed part are simultaneously formed. In this case, by using a heat block metal mold having plural (2 or more) flat heat sealing treatment surfaces, two or more flat heat-sealed parts can be simultaneously formed. When the flat heat-sealed parts and the embossed heat-sealed part are sequentially formed, heat block metal molds each having a heat sealing treatment surface corresponding to respective flat heat-sealed part or the embossed heat-sealed part is used. In this case, the width and shape of each heat-sealed part can be freely set according to the shape of the heat sealing treatment surface. Furthermore, when plural packages are to be simultaneously produced, treatment surfaces in the number corresponding to that of the packages to be produced may be formed on the heat block metal mold. Thereafter, a packaging film is punched with a punching edge to complete four-sided seal packages.
In the box motion method, a heat sealing treatment in the above-mentioned butt-sealing method is performed while continuously transporting a packaging film.

For production of the patch package of the present invention, either the butt-sealing method or the box motion method is preferably used to suppress development of pinholes as far as possible. When the patch package has a quadrate planar shape, a heat sealing treatment simultaneously forming heat-sealed parts on four sides is most preferable.

In the production of the patch package of the present invention, two or more flat heat-sealed parts may be simultaneously formed or sequentially formed. When they are simultaneously formed, however, the heat sealing treatment tends to be imcomplete due to dispersed pressure during the heat sealing treatment, leading to higher possibility of development of pinholes. Thus, the sequential formation is preferable. When two or more flat heat-sealed parts are formed sequentially, the order of formation is not particularly limited. However, the formation from the outer periphery side to the central part side of the package is preferable.

While the conditions of pressure bonding temperature, pressure bonding pressure, pressure bonding time and the like in the above-mentioned heat sealing treatment vary depending on the constitution of the packaging film (e.g., thickness of packaging film, kind of thermally adhesive resin constituting thermally adhesive resin layer etc.) and the like, they can be set as appropriate so as to obtain a desired band width and a desired seal strength. The pressure bonding temperature is generally within the range of 100°C to 250°C. When the pressure bonding temperature is lower than 100°C, the thermally adhesive resin layer does not thermally adhere, and when the pressure bonding temperature is higher than 250°C, the appearance of the resulting package tends to be poor due to the developments of wrinkles and bubbles, deformation and deterioration of the substrate film constituting the packaging film, and the like. The pressure bonding pressure is generally 0.2 MPa - 2 MPa, preferably 0.3 MPa - 1.5 MPa. When the pressure bonding pressure is lower than 0.2 MPa, pinholes are easily developed, and an appropriate seal strength tends to be difficult to achieve. In addition, when the pressure bonding pressure is higher than 2 MPa, a packaging film is deteriorated easily.

### (packaging film)

The packaging film of the packaging structure of the present invention is not particularly limited as long as it can form a heat-sealed part by a heat sealing treatment and can tightly seal the patch. Preferably, it has a substrate film layer and a thermally adhesive resin layer laminated on one side of the substrate film layer. The packaging film optionally has a dry lamination adhesive layer between the substrate film and the thermally adhesive resin layer to adhere them to each other. When the substrate film and the thermally adhesive resin layer are sufficiently adhered by thermal adhesion, the dry lamination adhesive layer may not be present.

As the substrate film layer, a film or sheet of a resin, which is superior in the mechanical, physical, chemical and other properties, particularly has a sufficient intensity and strength and heat resistance, and is superior in transparency, can be used. Specifically, for example, a film or sheet of one or more kinds of resin selected from polyester resins, polyamide resins, polyaramid resins, polyolefin resins such as polyethylene, polypropylene and the like, polystyrene resins, polyacrylic and polymethacrylic resins, polycarbonate resins, polyacetal resins, fluororesins, polyacrylonitrile resins, polyvinyl alcohol resins and the like can be mentioned. As such film or sheet of the above-mentioned resins, a film stretched in a monoaxial direction of the longitudinal direction or transverse direction can be used. Examples of the stretching method include known methods such as a flat method, an inflation method and the like, and the draw ratio thereof is about 2 - 10 fold. The thickness of the film is generally 5 µm - 100 µm, preferably, 10 µm - 50 µm. In the present invention, the above-mentioned resin film may be applied with a forward printing or reverse printing, and the like of a desired printed picture such as character, graphic, symbol, picture, pattern and the like by a general printing method.

As the dry lamination adhesive layer, a conventional dry lamination adhesive can be used, and a two-pack-curable adhesive is preferably used. Particularly, polyester urethane polyol/aromatic polyisocyanate adhesives, polyetherpolyurethane/epoxi adhesives, polyetherurethane polyol/aliphatic polyisocyanate adhesives, polyester/aliphatic isocyanate adhesives, polyester/aromatic isocyanate adhesives are more preferable, and polyester urethane polyol/aromatic polyisocyanate adhesives are still more preferable. To color a packaging film, a suitable amount of any pigment or dye can be added to a dry lamination adhesive layer.

A dry lamination adhesive layer can be formed by printing or coating a solution of a dry lamination adhesive to a surface of a substrate film layer or a thermally adhesive resin layer by gravure coating, roll coating, knife coating, spray coating, gravure printing, flexo printing, screen printing, offset printing, or other general printing method or coating method, and drying the printed or coating film. The thickness of the dry lamination adhesive layer is generally 1.0 g/m² - 30 g/m², preferably 2.0 g/m² - 20 g/m².

As a resin constituting the thermally adhesive resin layer, a resin that can be melted with heat and adhered to each other can be used. Specifically, for example, low density polyethylene, intermediate density polyethylene, high density polyethylene, linear low density polyethylene, polypropylene, ethylene-vinyl acetate copolymer, ionomer resin, ethylene-acrylic acid copolymer, ethylene-ethyl acrylate copolymer, ethylene-methacrylic acid copolymer, ethylene-methyl methacrylate copolymer, ethylene-propylene copolymer, methylpentene polymer, polybutene polymer, acid denatured polyolefin resin (polyolefin resin such as polyethylene, polypropylene and the like denatured with unsaturated carboxylic acid such as acrylic acid, methacrylic acid, maleic acid, fumaric acid, maleic anhydride and the like), polyvinyl acetate resin, poly(meth)acrylic resin, polyvinyl chloride resin, polystyrene resin, acrylonitrile resin (e.g., acrylonitrile-styrene copolymer (AS resin), acrylonitrile-butadiene-styrene copolymer (ABS resin) etc.), thermally adhesive poly(ethylene terephthalate) resin and the like can be used. Thermally adhesive resins most preferably used in the present invention include polyolefin resins and acrylonitrile resins such as low density polyethylene, intermediate density polyethylene, high density polyethylene, linear low density polyethylene, polypropylene and the like. A film or sheet made from the above-mentioned resins, or a coating film of a resin composition containing the above-mentioned resins as a main component and the like can constitute the thermally adhesive resin layer. The thickness thereof is generally 1 µm - 50 µm. When applied to a patch containing a drug, it is preferably 3 µm - 40 µm, more preferably 5 µm - 30 µm. When the thickness of the thermally adhesive resin layer is smaller than 1 µm, heat sealing property is degraded to permit easy development of pinholes, and when it is thicker than 50 µm, the drug in the patch is transferred into or adsorbed to a thermally adhesive resin layer at a higher proportion.

The packaging film of the present invention can have a middle substrate layer as necessary. Examples of the substrate constituting the middle substrate layer include materials that do not allow permeation of water vapor, water, gas and the like, and the like, which may be a single substrate or a composite substrate made of a combination of two or more kinds of substrates. Specifically, for example, a resin film having a vapor deposition film of an inorganic oxide such as silicon oxide, aluminum oxide and the like, which shows barrier property against water vapor, gas and the like; a film or sheet of a resin such as low density polyethylene, intermediate density polyethylene, high density polyethylene, linear low density polyethylene, polypropylene, ethylene-propylene copolymer and the like, which shows barrier property against water vapor, water and the like; a film or sheet of a resin such as polyvinylidene chloride, polyvinyl alcohol, ethylene-vinyl acetate copolymer saponified product and the like, which shows gas barrier property; a resin film having a vapor deposition film of aluminum; aluminum foil and the like can be used. While the thickness of the above-mentioned film or sheet can be appropriately set, it is generally 5 µm - 300 µm, preferably 10 µm - 100 µm. The thickness of the above-mentioned vapor deposition film of inorganic oxide is preferably 10 nm - 300 nm, and the thickness of the above-mentioned aluminum vapor deposition film is preferably about 10 nm - 40 nm. Examples of the resin film supporting the above-mentioned vapor deposition film include a polyester film, a polyamide film, a polyolefin film, a polyvinyl chloride film, a polycarbonate film, a polyvinylidene chloride film, a polyvinyl alcohol film, an ethylene-vinyl acetate copolymer saponified product film and the like. The thickness of the aluminum foil is preferably 3 µm - 30 µm, more preferably 5 µm - 10 µm.

A patch package using a packaging film made of an aluminum vapor deposition film or an aluminum foil as a middle substrate layer does not permit clear identification of the patch from the outside. Thus, the patch in the package cannot be tested with ease, and the presence or absence of a pinhole in the flat heat-sealed part cannot be confirmed with ease by visual inspection or image inspection. For packages difficult for appearance tests, ensured heat sealing during production is more important. In this sense, the patch package of the present invention is advantageous.

The packaging film in the patch package of the present invention is preferably transparent, since identification of the packed patch from the outside and confirmation of the presence or absence of a pinhole in the flat heat-sealed part are facilitated. Furthermore, it is preferably transparent, since the heat-sealed part of the packaging film can be clearly distinguished from other parts based on the transmission of light and difference in the refractive index (state of formation of heat-sealed part can be easily judged visually during heat sealing treatment). The flat heat-sealed part has higher transparency than other parts, since no gap is present between the upper and lower films and scattering of light can be suppressed. Thus, when the flat heat-sealed part is formed without a pinhole, the flat heat-sealed part can be considered to extend continuously. Furthermore, the presence or absence of a pinhole can be automatically examined by photographing the packaging structure and processing (binarizing) the images.

### (acrylonitrile resin layer)

When the packaging film of the present invention packs a patch containing a drug (i.e., "patch preparation"), the thermally adhesive resin layer is preferably an acrylonitrile type resin film layer (hereinafter sometimes to be referred to as an acrylonitrile type resin layer), since adsorption and passage of the drug is small and the content can be preserved well. The acrylonitrile resin used for the acrylonitrile type resin layer in the present invention is not particularly limited as long as not less than 50 wt% per total resin weight is comprised of acrylonitrile components. Thus, examples of the acrylonitrile resin include (i) polyacrylonitrile, (ii) a mixture or polymer alloy of polyacrylonitrile and other resin, (iii) a copolymer containing acrylonitrile as a main structural unit, (iv) a composition of a combination of at least two of the aforementioned (i) - (iii), and the like. Of these, any of the above-mentioned (ii) - (iv) is preferable in view of the suitable flexibility and rigidity as a packaging material of a patch, as well as heat sealability. Here, "acrylonitrile as a main structural unit" means that not less than 50 wt% of the total copolymer weight is acrylonitrile.

Acrylonitrile imparts non-adsorbability and gas barrier property to the acrylonitrile type resin layer, and adds rigidity to confer easy-to-tear property. Thus, the acrylonitrile type resin may be 100 wt% comprised of the acrylonitrile component. However, to achieve desired elasticity, impact resistance, tensile strength and the like of a package, a rubber component such as butadiene and the like and/or a (meth)acrylic acid alkyl ester component wherein the alkyl group has a carbon number of 1 - 6, and the like are preferably contained along with not less than 50 wt% of an acrylonitrile component. The rubber component imparts impact absorbability and suitable flexibility to acrylonitrile type resins, and the (meth)acrylic acid alkyl ester component lowers the melting point of the acrylonitrile type resin layer to improve heat sealability. In the (meth)acrylic acid alkyl ester wherein the alkyl group has a carbon number of 1 - 6, the alkyl group may be a straight chain or branched, and one or more kinds thereof can be used. Preferably, for example, methylacrylate, methylmethacrylate, ethylacrylate, ethylmethacrylate, (1- or 2-)propylacrylate, (1- or 2- )propylmethacrylate, (1- or 2-)butylacrylate, (1- or 2- )butylmethacrylate and the like can be mentioned. Of these, methylacrylate is particularly preferable, since it has broad utility, and can easily impart flexibility to a packaging material of a patch.

The acrylonitrile type resin is preferably a copolymer comprising acrylonitrile as a main structural unit since it affords uniform property of acrylonitrile type resin layer. The copolymer preferably contains, as a structural unit, at least acrylonitrile, a rubber component such as butadiene and the like and/or a (meth)acrylic acid alkyl ester wherein the alkyl group has a carbon number of 1 - 6. The copolymer preferably has an acrylonitrile content of 50 - 90 wt%, and a particularly preferable copolymer composition contains 50 - 90 wt% of acrylonitrile, 2 - 12 wt% of a rubber component such as butadiene and the like, and 8 - 38 wt% of a (meth)acrylic acid alkyl ester wherein the alkyl group has a carbon number of 1 - 6. In addition, the form of copolymerization of the copolymer is not particularly limited, and it may be random copolymerization, block copolymerization, or graft copolymerization. Of these, graft copolymerization is preferable, since it efficiently affords both the characteristics of polyacrylonitrile showing superior non-adsorbability of physiologically active components, and the characteristics of a rubber component having superior impact absorbability. As long as the characteristics are not inhibited, random copolymerization and block copolymerization may be added as appropriate.

The composition of the acrylonitrile type resin in the present invention is analyzed by the following method.

### (acrylonitrile content)

The acrylonitrile content is obtained by cutting out an acrylonitrile type resin layer from a packaging material, subjecting the sample to CHN elemental analysis and calculating the content from the nitrogen content. Furthermore, the molecular structure and weight ratio of the rubber component and the (meth)acrylic acid alkyl ester component wherein the alkyl group has a carbon number of 1 - 6 are determined from the ¹H-NMR and ¹³C-NMR (in deuterated DMSO, 80°C) spectrum ratio, and the weight ratio of 3 components is determined.

### (thickness of acrylonitrile type resin layer)

The thickness of the acrylonitrile type resin layer is appropriately set according to the kind and the like of the patch and is not particularly limited. To ensure nonpermeability and nonadsorbability of the physiologically active component in the patch, moisture permeability and suitable flexibility and rigidity of the layer as a packaging material of the patch, the thickness is preferably 10 µm - 100 µm, more preferably 10 µm - 80 µm, most preferably 10 µm - 50 µm.

### (patch)

While the patch in the present invention is not particularly limited, it is preferably adhered to the skin or mucosa to treat or prevent diseases. More preferably, it has an adhesive layer, a support laminated on one surface of the adhesive layer, and a release-treated liner laminated on the other surface of the adhesive layer. The adhesive layer may contain a transdermally absorbable drug.

As an adhesive constituting the adhesive layer, one that can be adhered to the skin or mucosa for a given time period, such as one showing adhesiveness in a normal state, one showing adhesiveness under moistening conditions, one showing adhesiveness under heating and the like can be used. To be specific, water-soluble adhesive base mainly containing polyvinyl alcohol, polyvinylpyrrolidone, poly-N-vinyl acetamide, polyethylene oxide, polyacrylamide, polyacryl acid salt, agar, xanthan gum and the like, water-swellable adhesive base mainly containing crosslinked water-soluble polymer, ethylcellulose, hydroxypropylcellulose, alginic acid ester and the like, and water-nonsoluble adhesive base mainly containing acrylic polymer, rubber polymer, silicone polymer, vinylether polymer, vinylester polymer and the like can be used. These adhesives and adhesive bases may contain, as necessary, known additives such as tackifier (e.g., rosin, denatured rosin, petroleum resin, polyterpene resin, polystyrene resin, polybutene resin, liquid polyisobutylene, glycerol and the like), plasticizer (e.g., liquid paraffin, fatty acid ester and the like), absorption promoter, surfactant, filler, water and the like to improve adhesiveness.

The thickness of the adhesive layer is generally 10 µm - 200 µm, preferably 15 µm - 150 µm.

When a patch preparation containing a drug in an adhesive layer is obtained, the drug is not particularly limited as long as it can be contained in an adhesive or an adhesive base in a dissolution state, supersaturated crystallization state or dispersion state, and the adhesive or adhesive base maintains adhesiveness. For example, corticosteroids, analgesic anti-inflammatory agent, sedative hypnotic, tranquilizer, antihypertensive agent, hypotensive diuretic, antibiotic, systemic anesthetic, antibacterial agent, antifungal agent, vitamin, coronary vasodilator, antihistamine agent, antitussive, sex hormone, antidepressant, brain circulation improver, antiemetic, antitumor agent, biological drug and the like can be mentioned.

While the content of the drug in an adhesive layer can be appropriately set according to the kind of the drug, it is generally 1 wt% - 80 wt%, preferably about 2 wt% - 70 wt%. When the content is less than 1 wt%, drug release in an amount effective for the treatment or prophylaxis cannot be expected. When it exceeds 80 wt%, adhesiveness decreases to prevent sufficient adhesion, which limits a theraprutic or prophylactic effect and is economically disadvantageous.

The support layer is not particularly limited as long as it does not produce a feeling of strangeness. Specifically, a single film or laminate film (solid film) made from one or more kinds of synthetic resins selected from polyester, polyolefin (polyethylene, polypropylene and the like), polyvinyl chloride, plasticized polyvinyl chloride, plasticized vinyl acetate-vinyl chloride copolymer, polyvinylidene chloride, ethylene-vinyl acetate copolymer, acetyl cellulose, ethylcellulose, ethylene-ethyl acrylate copolymer, polytetrafluoroethylene, polyurethane, ionomer resin and the like can be mentioned. In addition, porous film or sheet, non-woven fabric, woven fabric and the like, which are made from rubber, the above-mentioned synthetic resin, polyester (e.g., poly(ethylene terephthalate) etc.), polyamide (nylon etc.), can be mentioned. Moreover, laminates of these porous film or sheet, non-woven fabric, woven fabric and the like on a single film or laminate film made from the above-mentioned synthetic resins (composite film) can be mentioned. The thickness of the support layer is generally 1 µm - 1000 µm. In the case of a single film or laminate film made from the above-mentioned synthetic resins, the thickness is preferably 1 µm - 100 µm, and in the case of the above-mentioned composite film, it is preferably 100 µm - 1000 µm. The materials constituting the support layer in the present invention are not limited to the above-mentioned water insoluble materials, and water-soluble materials such as polyvinyl alcohol, polyvinylpyrrolidone, poly-N-vinylacetamide, polyethylene oxide, polyacrylamide, polyacryl acid salt, agar, xanthan gum and the like may also be used as long as they are nontoxic.

The exposed surface of the adhesive layer of the patch of the present invention is desirably covered and protected by a release liner until immediately before adhesion to the skin surface, to prevent adhesion of the adhesive layer to instruments, container and the like during production, adhesion of the adhesive layer to the packaging film during transport or preservation, and degradation of the patch. When in use, the release liner is detached to expose the surface of the adhesive layer, which is then adhered to the target site for administration. The release liner is not particularly limited as long as it is detached easily from the adhesive layer when in use. For example, a film of polyester, polyvinyl chloride, polyvinylidene chloride, poly(ethylene terephthalate) and the like, paper such as high-quality paper, glassine paper and the like, or a laminate film of high-quality paper or glassine paper etc. and polyolefin and the like, which has undergone a detach treatment by application of a silicone resin, fluororesin and the like to the surface to be in contact with the adhesive layer, are used. The thickness of the release liner is generally 10 µm - 200 µm, preferably 50 µm - 100 µm.

The production method of the patch is not particularly limited and, for example, a method including dissolving or dispersing a drug, an adhesive and the like in a solvent, applying the obtained solution or dispersion to one surface of a support, drying the support to form an adhesive layer on the surface of the support, laminating a release liner on the adhesive layer and the like can be mentioned. In addition, the patch can be produced by applying the above-mentioned solution or dispersion to a protective release liner, drying the release liner to form an adhesive layer thereon and adhering the support to the adhesive layer.

The total thickness of the patch to be packed is generally 50 µm - 2000 µm, preferably 100 µm - 1000 µm. When the thickness of the patch is less than 50 µm, the development of a pinhole can also be suppressed even by producing a patch package by a conventional technique. Therefore, the present invention is particularly useful when the thickness of the patch is not less than 50 µm. When the thickness of the patch is more than 2000 µm, distortion of the packaging film due to the thickness cannot be suppressed in the flat heat-sealed part, and pinholes are developed easily.

### EXAMPLES

The present invention is explained in detail in the following by referring to Examples and Comparative Examples, which are not to be construed as limitative. In the following, "part" and "%" mean "parts by weight" and "wt%", respectively.

### (preparation of sample)

Under an inert gas atmosphere, acrylic acid 2-ethylhexyl (75 parts), N-vinyl-2-pyrrolidone (22 parts), acrylic acid (3 parts) and azobisisobutyronitrile (0.2 part) in the adhesive solid content (100 parts by weight) were subjected to solution polymerization in ethyl acetate at 60°C to give a solution of an acrylic adhesive in ethyl acetate (adhesive solid content: 28%). Then, 80.3 kg of the solution of an acrylic adhesive in ethyl acetate and 27.8 kg of isopropyl myristate were mixed, and the viscosity was adjusted with ethyl acetate to give a coating solution. The obtained coating solution was applied to a poly(ethylene terephthalate) (hereinafter to be referred to as PET) release liner such that the thickness after drying was 150 µm and dried to form an adhesive film layer on the release liner. The adhesive layer of the release liner was adhered to a PET non-woven fabric surface of a PET support comprised of a laminate film of a PET non-woven fabric and a PET film, and the resulting product was preserved at 70°C for 48 hr to give an original coated fabric having a 4 layer constitution of PET film/adhesive layer/PET non-woven fabric/PET film. The original coated fabric was cut out in a 63.5 mm x 63.5 mm square (all corners had roundness (radius 6 mm)) to give a patch.

As a packaging film, a laminate film of transparent poly(ethylene terephthalate) film (thickness: about 12 µm)/aluminium foil (thickness: about 15 µm)/acrylonitrile type resin film (copolymer of acrylonitrile, butadiene and methylacrylate, thickness: about 30 µm) was used. A patch was contained, and a flat heat sealing treatment and an embossed heat sealing treatment were performed under the conditions shown in the following Table 1 (common treatment conditions) and Table 2 (separate treatment conditions) to tightly seal the packaging films, whereby the patch packages of Examples 1 and 2, and Comparative Examples 1 - 3 were obtained.
The flat heat-sealed part and embossed heat-sealed part were formed in a band-like shape, in a pattern surrounding the entire pheriphery of the patch. The size of the obtained patch packages was a 94 mm x 94 mm square (all corners had roundness (radius 5 mm)).

**Table 1**

| item | set value |
|---|---|
| sealing temperature (up/down) (°C) | 150/150 |
| pressure bonding time [sec] | 0.277 |
| pressure bonding pressure [MPa] | 0.33 |
| embossed heat sealing width [mm] | 45 |
| straw mat pattern pitch [mm] | 0.6 |
| straw mat pattern height [mm] | 0.3 |
| heat sealing treatment method | butt-sealing method |
| packaging machine | four-sided seal packaging machine |

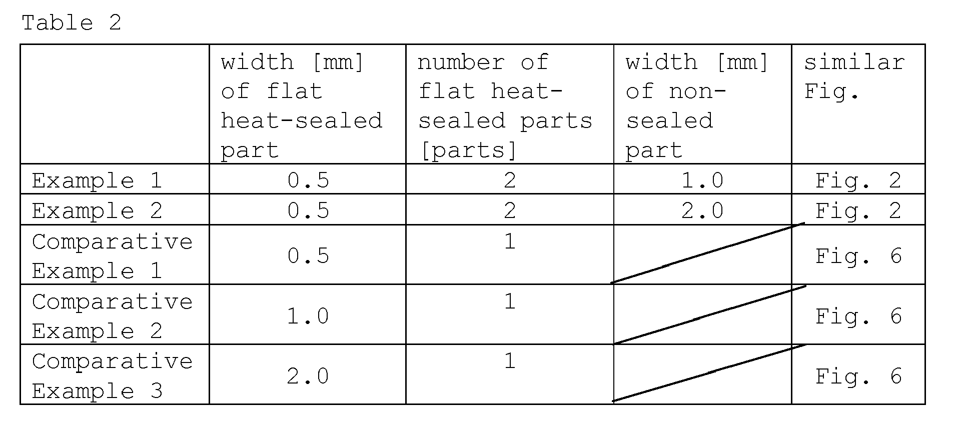

### (air-tightness)

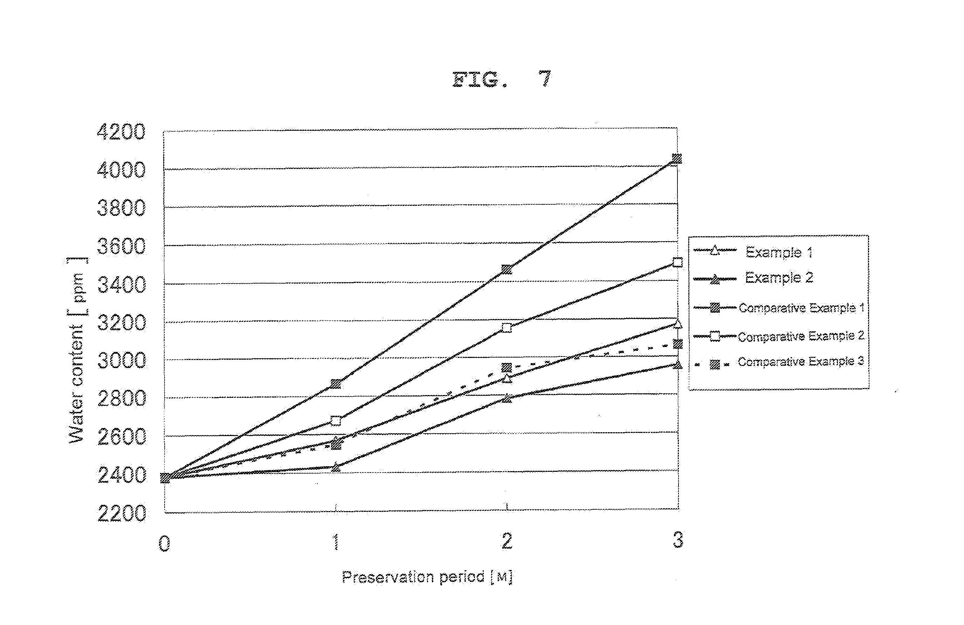

The patch packages of Examples 1 and 2, Comparative Examples 1 - 3 were preserved at 40±2°C and under relative humidity of 75±5% for 3 months, and the water content [ppm] of each patch was measured at the start of preservation, and 1 month, 2 months and 3 months later. The measurement method of the water content of the patch was as follows. The package was opened, the patch was removed and the release liner was removed to give a test piece, which was cast in a moisture vaporization apparatus. The above-mentioned treatment was completed immediately after opening of the package. The test piece was heated at 140°C in the moisture vaporization apparatus, the moisture generated thereby was introduced into a titration flask with nitrogen as a carrier, and the water content (ppm; weight ratio of moisture to total weight of test piece) of the test piece was measured by the Karl Fischer coulometric titration method.
The results are shown in Table 3 and Fig. 7.

**Table 3**

| | preservation start time | 1 month later | 2 months later | 3 months later |
|---|---|---|---|---|
| Example 1 | 2381 | 2569 | 2890 | 3172 |
| Example 2 | | 2432 | 2783 | 2957 |
| Comparative Example 1 | | 2864 | 3462 | 4038 |
| Comparative Example 2 | | 2672 | 3155 | 3494 |
| Comparative Example 3 | | 2547 | 2943 | 3062 |

The water content of the patches tightly sealed in the patch packages of Examples 1 and 2 (total width of flat heat-sealed part: 1.0 mm) were lower than not only the water content of the patch tightly sealed in a conventional patch package (width of flat heat-sealed part: 0.5 mm, Comparative Example 1) but also that of the patch tightly sealed in the patch package of Comparative Example 2 wherein the width of the flat heat-sealed part was 2 times (1.0 mm) that of conventional patch packages, and of the same level as that of the patch tightly sealed in the patch package of Comparative Example 3 wherein the width of the flat heat-sealed part was 4 times (2.0 mm) that of conventional patch packages. The results of Table 3 show superiority of the packaging structure of the present invention having two thin flat heat-sealed parts in the sealability to conventional packaging structures having only one thick (larger width) flat heat-sealed part, since the two thin flat heat-sealed parts are certainly formed at a uniform seal width and, even if one of them develops pinholes, the other flat heat-sealed part can suppress the leakage. As is clear from such results, the packaging structure of the present invention can retain superior air-tightness for a long time.

### (appearance of flat heat-sealed part)

The presence or absence of bubbles in the flat heat-sealed parts of the patch packages of Examples 1 and 2, and Comparative Examples 1 - 3 was evaluated. The absence or presence of bubble was visually evaluated.
The results are shown in Table 4.

**[Table 4]**

| | number of evaluation | bubble | no bubble |
|---|---|---|---|
| Example 1 | 10 | 0 | 10 |
| Example 2 | | 0 | 10 |
| Comparative Example 1 | | 0 | 10 |
| Comparative Example 2 | | 2 | 8 |
| Comparative Example 3 | | 10 | 0 |

The flat heat-sealed parts of the patch packages of Examples 1 and 2 did not show a bubble in any of 10 samples. In contrast, the flat heat-sealed parts of the patch packages of Comparative Example 2 showed a bubble in 2 out of 10 samples, and the patch package of Comparative Example 3 showed a bubble in all 10 samples. The results of Table 4 show that a pinhole failure is easily developed when the width of flat heat sealing is not less than 1 mm. Therefore, when only one flat heat-sealed part having a width of 1 mm is formed, the sealability may not be ensured. As is clear from such results, the packaging structure of the present invention solves the sealing defects due to pinholes, and can form a beautiful flat heat-sealed part.

As is clear from the Examples, the patch package of the present invention is free of pinholes, superior in air-tightness, and has good appearance of the flat heat-sealed part. Particularly, the patch package is highly useful when a patch contains a drug easily decomposed by oxygen or moisture, since it can highly suppress oxidation and decomposition due to its long-term preservation.

## Claims

1. A patch package comprising a patch and a packaging film sandwiching the patch, wherein the packaging film is tightly sealed in two or more flat heat-sealed parts, and respective adjacent flat heat-sealed parts of the two or more flat heat-sealed parts are separated across a non-sealed part.

2. The patch package of claim 1, further comprising an embossed heat-sealed part outside the outermost flat heat-sealed part of the two or more flat heat-sealed parts.

3. The patch package of claim 2, wherein the outermost flat heat-sealed part and the embossed heat-sealed part are separated across a non-sealed part.

4. The patch package of any one of claims 1 to 3, wherein the two or more flat heat-sealed parts, or the two or more flat heat-sealed parts and the embossed heat-sealed part form a pattern surrounding the patch.

5. The patch package of any one of claims 1 to 4, wherein the patch is sandwiched between two packaging films, and the two or more flat heat-sealed parts, or the two or more flat heat-sealed parts and the embossed heat-sealed part form a pattern surrounding the entire periphery of the patch.

6. The patch package of any one of claims 1 to 4, wherein the patch is sandwiched by a double-folded packaging film, and the two or more flat heat-sealed parts, or the two or more flat heat-sealed parts and the embossed heat-sealed part are not formed in a bent part of the packaging film.

7. The patch package of any one of claims 1 to 6, wherein each of the two or more flat heat-sealed parts has a band-like

8. The patch package of claim 7, wherein the band-like flat heat-sealed parts have a band width of 0.4 mm - 1.0 mm.

9. The patch package of any one of claims 1 to 8, wherein the non-sealed part has a band-like shape and has a band width of 1.5 mm - 10 mm.

10. The patch package of any one of claims 1 to 9, having three flat heat-sealed parts, or three flat heat-sealed parts and an embossed heat-sealed part.

11. The patch package of any one of claims 1 to 10, wherein the patch is a patch preparation containing a drug.
